(19) Europäisches Patentamt
European Patent Office
Office européen des brevets

(11) Publication number: **0 197 013**
**A1**

(12) # EUROPEAN PATENT APPLICATION

(21) Application number: **86850050.5**

(22) Date of filing: **17.02.86**

(51) Int. Cl.⁴: **C 07 D 401/12**
A 61 K 31/415, A 61 K 31/44

(30) Priority: **01.03.85 SE 8500996**

(43) Date of publication of application:
**08.10.86 Bulletin 86/41**

(84) Designated Contracting States:
**AT BE CH DE FR GB IT LI LU NL SE**

(71) Applicant: **Aktiebolaget Hässle**
**Kärragatan 5**
**S-431 83 Mölndal(SE)**

(72) Inventor: **Brändström, Arne Elof**
**Anders Mattssonsgatan 13B**
**S-415 06 Göteborg(SE)**

(72) Inventor: **Lindberg, Per Lennart**
**Knapehall 64**
**S-436 00 Askim(SE)**

(72) Inventor: **Sunden, Gunnel Elisabeth**
**Eketrägatan 24A**
**S-417 12 Göteborg(SE)**

(72) Inventor: **Wallmark, Björn Morgan Gabriel**
**Rada Portar 97**
**S-435 00 Mölnlycke(SE)**

(74) Representative: **Hjertman, Ivan T. et al,**
**AB ASTRA Patent and Trade Mark Department**
**S-151 85 Södertälje(SE)**

(54) **Substituted benzimidazoles for the manufacture of a medicament for treatment of intestinal inflammatory diseases.**

(57) A method for the treatment of intestinal diseases such as cholera, paratyphoid, tourist diarrhoea, toxin-induced diarrhoea, ulcerous colitis and regional enteritis comprising administering to a patient suffering therefrom an amount of a compound of the formula I

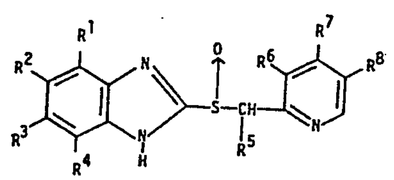

I

or a pharmaceutically acceptable salt thereof, effective to mitigate the symptoms of the intestinal disease.

EP 0 197 013 A1

Croydon Printing Company Ltd.

0197013

# Substituted benzimidazoles for the manufacture of a medicament for treatment of intestinal inflammatory diseases

## Field of the Invention

The present invention is related to a method for the treatment of intestinal diseases, especially inflammatory intestinal diseases.

## Background of the Invention

Intestinal diseases are a great problem to mankind. Examples of intestinal diseases are cholera, paratyphoid, tourist diarrhoea, toxin-induced diarrhoea, ulcerous colitis and regional enteritis. In some bacterial diarrhoeas antibiotics or chemotherapeutics may be of value but in all severe cases treatment of fluid and electrolyte losses is the most important therapeutic principle. Symptomatic treatment with agents inhibiting the intestinal motility is usually of limited value.

Control of water and salt losses in patients with diarrhoea is at present obtained by the administration of oral and/or intravenous solutions of electrolytes. Drugs interfering with the transport of water and ions, like $Na^+$ and $K^+$, across the gastrointestinal mucosa will be of great value in treatment of this type of diseases especially in earlier stages in order to prevent serious fluid disturbances.

## Outline of the Invention

According to the present invention it has been found that the compounds with the general formula I

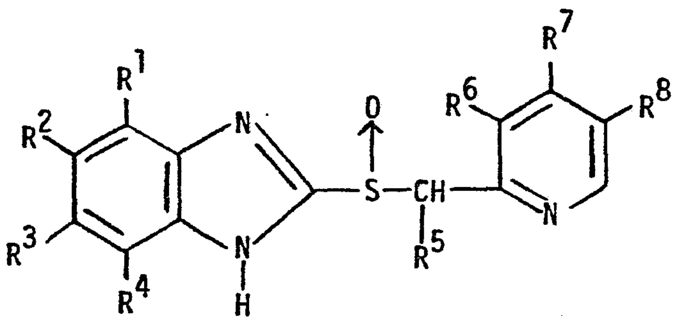

I

or a pharmaceutically acceptable salt thereof in which $R^1$, $R^2$, $R^3$ and $R^4$ are the same or different and are

hydrogen
alkyl containing 1-6 carbon atoms
alkoxy containing 1-6 carbon atoms
halogen, e.g. F, Cl, Br I
$-CF_3$
$-NO_2$
$-COR^9$ ($R^9$ is alkyl or cycloalkyl having 1-6 carbon atoms, aryl containing up to 10 carbon atoms optionally substituted by halogen, methyl or methoxy, or alkoxy containing 1-2 carbon atoms)

or wherein adjacent groups $R^1$, $R^2$, $R^3$ and $R^4$ together with the adjacent carbon atoms in the benzimidazole ring form a 5- or 6-membered ring containing 0-3 hetero atoms selected from N and O and which rings may be either saturated including one or two oxo groups $(-\overset{O}{\overset{\|}{C}}-)$ directly attached to the benzimidazole ring or unsaturated

$R^5$ is hydrogen

$R^6$ and $R^8$, which are the same or different are
hydrogen
methyl or
ethyl

$R^7$ is hydrogen
methyl
methoxy
ethoxy or
aryloxy, wherein the aryl contains up to 10 carbon atoms, which may be optionally substituted by alkyl containing 1-6 carbon atoms

provided that

a) when more than one of $R^6$, $R^7$ and $R^8$ are hydrogen and $R^7$ is other than aryloxy, then at least two of $R^1$, $R^2$, $R^3$ and $R^4$ are hydrogen;

b) when $R^7$ is methoxy or ethoxy and $R^6$ and $R^8$ are hydrogen, then $R^1$, $R^2$, $R^3$ and $R^4$ are other than CN, $NO_2$ or a 5- or 6-membered ring;

c) when $R^7$ is aryloxy then $R^1$, $R^2$, $R^3$ and $R^4$ cannot be -CN, $-NO_2$, -CO--aryl, $-COOCH_3$, $-COOC_2H_5$ or a 5- or 6-membered ring;

d) when more than one of $R^6$, $R^7$ and $R^8$ are hydrogen and one of $R^1$, $R^2$, $R^3$ and $R^4$ is $CF_3$, then $R^7$ is other than ethoxy;

e) when $R^7$ is methoxy, $R^6$ and $R^8$ are hydrogen and one of $R^1$, $R^2$, $R^3$ and $R^4$ is $CF_3$, then the others of $R^1$, $R^2$, $R^3$ and $R^4$ must be hydrogen.

Some of the substituted benzimidazoles with the general formula I are described in e.g. EP-A1-5129. Others are described in BE-A-898 880, EP-A-80602, US-A-4 182 766, US-A-4 045 563, EP-A-130729, DE-A-3 415 971 and SE 416 649. The compounds of the formula I, wherein $R^7$ is a group containing an aromatic ring are disclosed in the Swedish patent application 8404065-8, which not yet is available to the public. The last-mentioned compounds are prepared according to one of the following methods.

a) Oxidizing a compound of the formula II

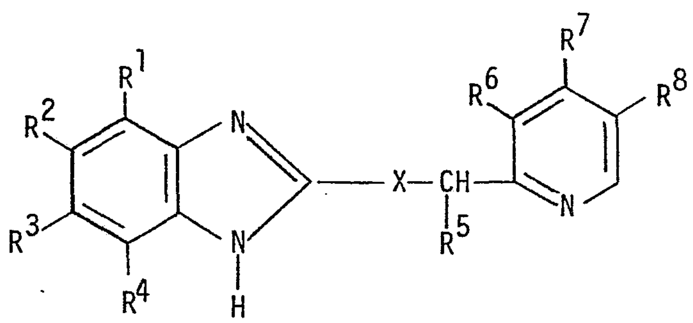

II

wherein X is S and $R^1$, $R^2$, $R^3$, $R^4$, $R^5$, $R^6$, $R^7$ and $R^8$ have the meanings given above, to give a compound of the formula I.

The oxidation may be carried out by using an oxidizing agent selected from the group consisting of nitric acid, hydrogen peroxide, peracids, peresters, ozone, dinitrogentetraoxide, iodosobenzene, N-halosuccinimide, 1-chlorobenzotriazole, t-butylhypochlorite, diazabicyclo-[2,2,2]-octane bromine complex, sodium metaperiodate, selenium dioxide, manganese dioxide, chromic acid, cericammonium nitrate, bromine, chlorine, and sulfuryl chloride. The oxidation usually takes place in a solvent wherein the oxidizing agent is present in some excess in relation to the compound to be oxidized.

The oxidation may also be carried out enzymatically by using an oxidating enzyme or microbiotically by using a suitable microorganism.

b) Hydrolyzing a compound of the formula III

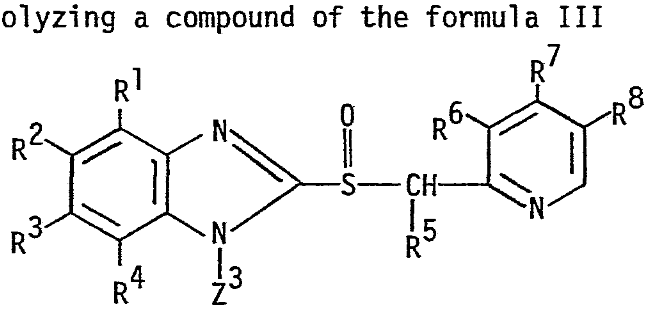

II

wherein $R^1$, $R^2$, $R^3$, $R^4$, $R^5$, $R^6$, $R^7$ and $R^8$ are as defined above and $Z^3$ is a suitable N-protecting group such as alkanoyl, carboalkoxy and trimethylsilyl, to the formation of a compound of the formula I.

The alkanoyl group in $Z^3$ can have 1-6 carbon atoms and the carboalkoxy group 2-6 carbon atoms. The hydrolysis may be performed in alkaline solution.

c) Reacting a compound of the formula IV

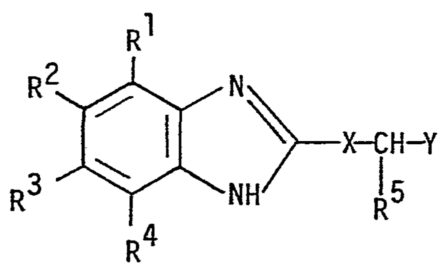

IV

wherein $R^1$, $R^2$, $R^3$, $R^4$ and $R^5$ are as defined above, X is -S- or -SO- and Y is K, Na or Li,

with a compound of the formula

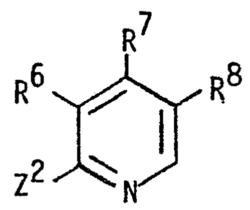

V

wherein $R^6$, $R^7$ and $R^8$ are as defined above and $Z^2$ is halogen such as Cl or Br;

whereafter the compound obtained, when X is -S-, is oxidized to a compound of the formula I.

The end products of the formula I are obtained as the free base.

The preparation is further illustrated by the following examples.

<u>Example 1.</u> Method a. Preparation of 5,6-dimethyl-2-[[(4-phenoxy-5--ethyl-2-pyridinyl)methyl]sulfinyl]-1*H*-benzimidazole.

m-Chloroperbenzoic acid, 90.4% (0.56 g, 0.0029 mol) dissolved in $CH_2Cl_2$ (15 ml) was added under stirring to a cooled (-2°C) solution of 5,6-dimethyl-2-[[(4-phenoxy-5-ethyl-2-pyridinyl)methyl]thio]-1*H*-benzimidazole (1.2 g, 0.0029 mol) in $CH_2Cl_2$ (50 ml) maintaining the temperature at -2°C. Stirring was continued at -2°C for 5 min and then $NaHCO_3$ (0.75 g, 0.0089 mol) dissolved in water (50 ml) was added under vigorous stirring. The two phases were separated and the $CH_2Cl_2$-phase was washed with water (20 ml). The organic phase was dried ($MgSO_4$) and the solvent was evaporated off. The residue was crystallized from $CH_3CN$ yielding the desired product (0.68 g, 57%), m.p. 146°C.

Example 2. Method a. Preparation of 5-methoxy-2-[[[4-(4-methylphenoxy)-2-pyridinyl]methyl]sulfinyl]-1H-benzimidazole.

m-Chloroperbenzoic acid, 82% (1.54 g, 0.0073 mol) dissolved in $CH_2Cl_2$ (20 ml) was added under stirring to a cooled (-5°C) solution of 5-methoxy-2-[[[4-(4-methylphenoxy)-2-pyridinyl]methyl]thio]-1H-benzimidazole (2.9 g, 0.0073 mol) in $CH_2Cl_2$ (50 ml) maintaining the temperature at -5°C. After the addition NaOH (0.87 g, 0.022 mol) dissolved in water (30 ml) was added under vigorous stirring. The two phases were separated. More $CH_2Cl_2$ was added to the aqueous phase, the pH was adjusted to 9.5 by adding 2M HCl and after stirring the phases were separated. The organic phase was dried ($Na_2SO_4$) and the solvent was evaporated off. The residue was crystallized from $CH_3CN$ yielding the desired product (1.2 g, 42%), m.p. 121°C.

The compounds are known to inhibit gastric acid secretion and may thus be used in the treatment of peptic ulcer disease.

The antisecretory activity of the substituted benzimidazoles with the general formula I has been found to be mediated by inhibition of the gastric $H^+,K^+$-ATPase, exerted by certain degradation products. This enzyme is responsible for the pumping of protons into the stomach.

Recently, a certain "colon $K^+$-ATPase" enzyme has been shown to be located in the distal part of colon. c.f. Gustin, M.C. and Goodman, D.B.P. (1981) J. Biol. Chem. 256, 10651-10656. The "colon $K^+$-ATPase" is situated in the apical brush border membrane of the colonic epithelium cell and is postulated to be responsible for the absorptive transmembrane movement of $K^+$ from the mucosal to the serosal side of the colon. This enzyme is thus believed to be of great importance for the electrolyte balance across the mucosal barrier in the colon.

The two enzymes gastric $H^+,K^+$-ATPase and colon $K^+$-ATPase appear to have similar but not the same way of acting. Gastric $H^+,K^+$-ATPase, which is located in the parietal cells, has a central role in the secretion of HCl by the gastric epithelium. There are, however, no evidence that protons are secreted by the colon.

As stated above the electrolytic balance is very often disturbed in diseases involving diarrhoeatic states. Thus compounds having an influence on the colonic $K^+$-ATPase enzyme may be expected to be therapeutically valuable in the treatment of diseases involving diarrhoea.

It has now surprisingly been found that also the colonic $K^+$-ATPase enzyme is strongly inhibited in vitro by the above-mentioned degradation products of the substituted benzimidazoles of the general formula I. Since such degradation products are formed, (with the rate of formation dependent on the substituents in the two aromatic rings as well as on the surrounding pH:es) during the in vivo passage of the gastrointestinal tract, the compounds of the general formula I have thus a therapeutic effect on diarrhoea diseases. Examples of such diseases are inflammatory intestinal diseases such as cholera, paratyphoid, tourist diarrhoea and toxin-induced diarrhoea but also other intestinal diseases such as ulcerous colitis and regional enteritis.

The substituted benzimidazoles with the formula I may be used either as the free base or their pharmaceutically acceptable salts, all of which are included within the scope of the invention. Thus, basic, neutral or mixed salts may be obtained as well as hemi, mono, sesqui or polyhydrates. In the preparation of alkaline salts the benzimidazole is reacted with NaOH, NaOR, $NaNH_2$, $NaNR_2$, $Mg(OR)_2$, LiOH, KOH, LiOR, $LiNH_2$, $LiNR_2$, KOR, $KNH_2$, $KNR_2$, $Ca(OR)_2$, $CaH_2$, $Ti(OR)_4$, $TiH_4$ or

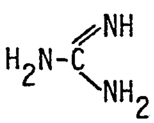 , wherein R is an alkyl group containing 1-4 carbon atoms.

Preferred compounds with the general formula I are compounds, wherein

I At least one of $R^1$, $R^2$, $R^3$ and $R^4$ is other than hydrogen and alkyl, wherein alkyl is as defined above;

II At least one of $R^1$, $R^2$, $R^3$ and $R^4$ is other than hydrogen, alkyl and alkoxy, wherein alkyl and alkoxy are as defined above;

III Not more than two of $R^1$, $R^2$, $R^3$ and $R^4$ are other than hydrogen, alkyl and alkoxy, wherein alkyl and alkoxy are as defined above;

IV Only one of $R^1$, $R^2$, $R^3$ and $R^4$ is other than hydrogen, alkyl and alkoxy, wherein alkyl and alkoxy are as defined above;

V Preferably $R^2$ is a group other than hydrogen, alkyl or alkoxy, wherein alkyl and alkoxy are as defined above;

VI Preferably $R^1$ is a group other than hydrogen, alkyl or alkoxy, wherein alkyl and alkoxy are as defined above;

VII Preferably $R^2$ is a group other than hydrogen, alkyl or alkoxy and $R^1$, $R^3$ and $R^4$ are hydrogen or alkyl, wherein alkyl and alkoxy are as defined above;

VIII Preferably $R^2$ is a group other than hydrogen, alkyl or alkoxy and $R^1$, $R^3$ and $R^4$ are hydrogen, wherein alkyl and alkoxy are as defined above;

Especially preferred benzimidazoles with the formula I are the compounds listed in the following Table 1, wherein the use of the first listed compound, omeprazole, is considered to be the best mode of carrying out the invention known at present.

Table 1.

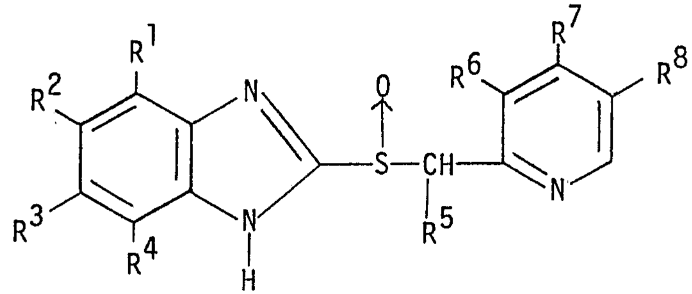

I

| $R^1$ | $R^2$ | $R^3$ | $R^4$ | $R^5$ | $R^6$ | $R^7$ | $R^8$ |
|---|---|---|---|---|---|---|---|
| H | $OCH_3$ | H | H | H | $CH_3$ | $OCH_3$ | $CH_3$ |
| H | CO– 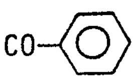 | H | H | H | $CH_3$ | $OCH_3$ | $CH_3$ |
| $CH_3$ | $COCH_3$ | $CH_3$ | H | H | $CH_3$ | $OCH_3$ | $CH_3$ |
| $CH_3$ | $COCH_3$ | $CH_3$ | H | H | $CH_3$ | H | $CH_3$ |
| $CH_3$ | $COC_2H_5$ | $CH_3$ | H | H | $CH_3$ | $OCH_3$ | $CH_3$ |
| $OCH_3$ | Br | $OCH_3$ | H | H | $CH_3$ | $OCH_3$ | $CH_3$ |
| $OCH_3$ | Br | $OCH_3$ | H | H | $CH_3$ | $CH_3$ | H |
| $C_2H_5$ | CN | $C_2H_5$ | H | H | $CH_3$ | $OCH_3$ | $CH_3$ |
| $C_2H_5$ | CN | $C_2H_5$ | H | H | $CH_3$ | $OC_2H_5$ | $CH_3$ |
| Cl | Cl | Cl | H | H | $CH_3$ | $OCH_3$ | $CH_3$ |
| $COCH_3$ | $CH_3$ | $CH_3$ | $CH_3$ | H | $CH_3$ | $OCH_3$ | $CH_3$ |
| F | Cl | H | Cl | H | $CH_3$ | $OCH_3$ | $CH_3$ |

cont.

Table 1. cont.

| $R^1$ | $R^2$ | $R^3$ | $R^4$ | $R^5$ | $R^6$ | $R^7$ | $R^8$ |
|---|---|---|---|---|---|---|---|
| $-CH=CH-CH=CH-$ | | $-CH=CH-CH=CH-$ | | H | $CH_3$ | $OCH_3$ | $CH_3$ |
| $-CH=CH-CH=N-$ | | H | H | H | $CH_3$ | $OCH_3$ | $CH_3$ |
| $-CH=CH-CH=CH-$ | | H | H | H | $CH_3$ | $OCH_3$ | $CH_3$ |
| H | $-CH=CH-CH=CH-$ | | H | H | $CH_3$ | $OCH_3$ | $CH_3$ |
| $CH_3$ | CN | $CH_3$ | H | H | $CH_3$ | $OC_2H_5$ | $CH_3$ |
| H | $COOCH_3$ | $CH_3$ | H | H | H | $OCH_3$ | $C_2H_5$ |
| H | $SOCH_3$ | H | H | H | $CH_3$ | $OCH_3$ | $CH_3$ |
| H | $NO_2$ | H | H | H | $CH_3$ | $OCH_3$ | $CH_3$ |
| H | $CF_3$ | H | H | H | H | 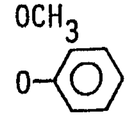 | $CH_3$ |
| H | Cl | H | H | H | H | 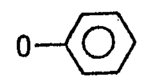 | H |
| H | $COCH_3$ | H | H | H | $CH_3$ | 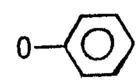 | H |
| H | $COCH_3$ | $CH_3$ | H | H | H | $CH_3$ | $CH_3$ |
| H | $COOCH_3$ | $CH_3$ | H | H | H | $CH_3$ | $CH_3$ |
| H | $COOCH_3$ | H | H | H | H | $CH_3$ | $CH_3$ |
| H | $COCH_3$ | $CH_3$ | H | H | $CH_3$ | $CH_3$ | H |
| H | $COOCH_3$ | $CH_3$ | H | H | $CH_3$ | $CH_3$ | H |

cont.

Table 1. cont.

| $R^1$ | $R^2$ | $R^3$ | $R^4$ | $R^5$ | $R^6$ | $R^7$ | $R^8$ |
|---|---|---|---|---|---|---|---|
| H | $COCH_3$ | $CH_3$ | H | H | $CH_3$ | H | $CH_3$ |
| H | $COCH_3$ | $CH_3$ | H | H | $CH_3$ | $CH_3$ | $CH_3$ |
| H | $COCH_3$ | $CH_3$ | H | H | H | $OCH_3$ | H |
| H | $COCH_3$ | $CH_3$ | H | H | $CH_3$ | $OCH_3$ | $CH_3$ |
| H | $COOCH_3$ | $CH_3$ | H | H | $CH_3$ | H | $CH_3$ |
| H | $COOCH_3$ | $CH_3$ | H | H | $CH_3$ | $CH_3$ | $CH_3$ |
| H | $COOCH_3$ | $CH_3$ | H | H | H | $OCH_3$ | H |
| H | $COOCH_3$ | $CH_3$ | H | H | H | $OC_2H_5$ | H |
| H | $COOCH_3$ | $CH_3$ | H | H | $CH_3$ | $OCH_3$ | H |
| H | $COOCH_3$ | $CH_3$ | H | H | $CH_3$ | $OCH_3$ | $CH_3$ |
| H | $COOCH_3$ | $CH_3$ | H | H | H | $OCH_3$ | $CH_3$ |
| H | $COOCH_3$ | H | H | H | $CH_3$ | H | $CH_3$ |
| H | $COOCH_3$ | H | H | H | $CH_3$ | $OCH_3$ | $CH_3$ |
| H | $COCH_3$ | H | H | H | $CH_3$ | $OCH_3$ | $CH_3$ |
| H | Cl | H | H | H | $CH_3$ | $OCH_3$ | $CH_3$ |
| H | $COOC_2H_5$ | H | H | H | $CH_3$ | $OCH_3$ | $CH_3$ |

cont.

| $R^1$ | $R^2$ | $R^3$ | $R^4$ | $R^5$ | $R^6$ | $R^7$ | $R^8$ |
|---|---|---|---|---|---|---|---|
| $CF_3$ | H | H | H | H | H | $OCH_3$ | H |
| $CF_3$ | H | H | H | H | $CH_3$ | $OCH_3$ | H |
| $CF_3$ | H | H | H | H | H | $OCH_3$ | $CH_3$ |
| $CF_3$ | H | H | H | H | $CH_3$ | $OCH_3$ | $CH_3$ |
| H | $CF_3$ | H | H | H | H | $OCH_3$ | H |
| H | $CF_3$ | H | H | H | $CH_3$ | $OCH_3$ | H |
| H | $CF_3$ | H | H | H | H | $OCH_3$ | $CH_3$ |
| H | $CF_3$ | H | H | H | $CH_3$ | $OCH_3$ | $CH_3$ |
| H | -CH=CH-CH=CH- | | H | H | H | H | $C_2H_5$ |
| $CH_3$ | H | Cl | H | H | H | H | H |
| H | $COCH_3$ | H | H | H | H | H | H |
| H | $COOC_2H_5$ | H | H | H | H | H | H |
| H | Br | H | H | H | H | H | H |
| H | Cl | H | H | H | H | H | $CH_3$ |
| H | $CF_3$ | H | H | H | H | H | H |
| H | Cl | H | H | H | H | H | H |
| Cl | H | H | H | H | H | H | H |

cont

Table 1. cont.

| $R^1$ | $R^2$ | $R^3$ | $R^4$ | $R^5$ | $R^6$ | $R^7$ | $R^8$ |
|---|---|---|---|---|---|---|---|
| H | -CO- 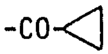 | H | H | H | H | $OCH_3$ | H |
| H | -CO- 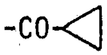 | H | H | H | H | $CH_3$ | H |
| H | -CO- 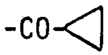 | H | H | H | H | $OCH_3$ | $CH_3$ |
| H | -CO- 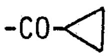 | H | H | H | $CH_3$ | H | H |
| H | -CO- 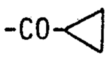 | H | H | H | $CH_3$ | $OCH_3$ | $CH_3$ |
| H | -CO- 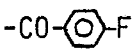 | H | H | H | H | $OCH_3$ | H |

Pharmacological tests

In order to evaluate the inhibitory effect on colonic $K^+$-ATPase of the substituted benzimidazoles with the general formula I, we have tested one compound, namely omeprazole on an enzyme preparation from the descending colon of the rabbit (cf. below).

In the case of omeprazole the most efficient way to form the active degradation products is to decompose it in acid solution. Thus, omeprazole was decomposed in acid before it was added to the enzyme.

EXPERIMENTAL PROCEDURES

Preparation from descending colon

In order to isolate and characterize the ouabain insensitive potassium-stimulated ATPase from rabbit descending colon the following preparation was made.

Apical brush-border membranes were prepared from rabbit descending colon epithelium, by the method of Gustin M.C., and Goodman D.B.P., J. Biol. Chem. 256, 10651-10656 (1981), with some modifications.

Two albino male rabbits, weighing 2-3 kg, were killed by injection of mebumal into the ear vein. A section of colon, 30-35 cm in length, measured from rectum, was removed, rinsed free of its content with icecold 0.15 M NaCl and everted over a glassrod. The tissue was then divided in two pieces. These everted segments were tied at one end, filled with an icecold buffer containing 30 mM NaCl, 5mM $Na_3$EDTA and 8 mM Hepes/Tris pH 7.6, using a 20 ml syringe without a needle, until they became distended, at which point the other end was tied. The intestines were transferred to a 500 ml centrifuge tube containing the same cold pH 7.6 buffer. The tube was placed in an icebox, which was shaken for 2 hours at 260 osc./min. Then the incubation medium was filtered through a nylon net of 200 µm mesh, to remove debris.

Sedimentation of epithelial cells was performed by centrifugation at 500xg for 10 minutes at 4°C, in a Beckman J2-21 centrifuge. The pelleted cells were resuspended in 250 ml of a solution containing 1mM $Na_3$EDTA, adjusted to pH 8.3 with HCl at 4°C, just before use. The cells were then disrupted, by a 28 second homogenization in a Waring Blendor. The resulting homogenate was centrifuged for 10 minutes at 4°C with a centrifugal force of 750xg in a Beckman J2-21 centrifuge with a JA-17 rotor, in order to sediment brush-borders. Pellets were combined, resuspended in 40 ml of the medium containing 1mM $Na_3$EDTA and recentrifuged as described above. The brush-border pellet ($P_2$) was resuspended in 33.3 ml of the same medium, 2.7 ml density gradient medium (Percoll, Pharmacia) was added by mixing. The Percoll medium was allowed to generate a density gradient during 22 minutes at 40 000xg in a Beckman J2-21 centrifuge, in which the membranes sedimented into bands. Nine fractions (G1-G9) of 4 ml each, were carefully removed from the gradient with a Pasteur-pipette. The fractions were subjected to centrifugation in a Kontron TGA-65 centrifuge with a TST 60 swing-out rotor at 50 000 rpm for 45 minutes, to sediment the Percoll. The material collected on the top of the Percoll pellet was homogenized by a glass-teflon homogenizer with 5-10 up-down strokes at about 1500 rpm.

Determination of ATPase activity

ATPase activity was determined as the amount of $P_i$ (free phosphate) liberated from ATP by the enzyme per unit of time.

The assay medium contained 2mM ATP, 2mM $MgCl_2$, 18.4 mM Pipes/Tris pH 7.4 and membrane protein in the presence or absence of 10 mM KCl in a final volume of 1 ml. In some experiments, $10^{-5}$ M of the specific $Na^+,K^+$-ATPase inhibitor ouabain was present. The enzyme $Na^+,K^+$-ATPase is responsible for the active transport of $Na^+$ out from the cell into the plasma as well as for the transport of $K^+$ into the cell from the plasma. Unless otherwise mentioned, the preparation was preincubated with ouabain on ice for 10 minutes before start of the reaction, which was initiated with ATP, mixed and incubated for suitable time at 37°C. The reaction was stopped by the addition of 1 ml icecold 12 per cent perchloric acid, containing 3.6 per cent ammoniummolybdate. 3 ml butylacetate was added and the testtubes were shaken on a mixer for 15 seconds in order to

extract the phosphomolybdate complex into the organic phase. The absorbance of this complex was measured at 320 nm against a reagent blank.

Inhibition by acid decomposed omeprazole

Omeprazole was dissolved in methanol to a concentration of 20 mM. 100 µl of this solution, was diluted to 10 ml with a buffer, which gave a final concentration of 0.2 mM omeprazole and 2 mM Na-Acetate/HCl, pH 5.0. A control medium was made in the same way, without omeprazole present. These media were treated for 120 minutes at 37°C. The reaction was quenched by addition of an equivalent volume of 150 mM Tris/HCl, pH 7.4. The control medium was treated in the same way.

10 µg colon apical membrane samples were incubated for 10 minutes at 37°C with the quenched solutions, giving the concentrations of omeprazole indicated in the result table, in final volumes of 1 ml. The $K^+$-stimulated ATPase activity was determined for 10 minutes.

Table

Effect of acid decomposed omeprazole on colon $K^+$-ATPase

| Initial concentration of omeprazole in the break-down mixture µM | Remaining enzyme activity % Mean value ± S.D. |
|---|---|
| 1.0 | 100.0 ± 5.9 |
| 2.5 | 72.4 ± 6.0 |
| 5.0 | 31.9 ± 6.7 |
| 7.5 | 18.6 ± 5.5 |
| 10.0 | 5.2 ± 3.2 |

This table shows that a mixture having a concentration of 10 µM almost completely inhibits the activity of the colonic $K^+$-ATPase enzyme. Thus, omeprazole may be used in the treatment of intestinal diseases, wherein the electrolyte balance across the mucosal barrier in the colon is disturbed.

Pharmaceutical preparations

In clinical use the compounds of the invention are administered orally, in the form of a pharmaceutical preparation which does not release the active sulphoxide compound before it reaches the colon and which contains the sulphoxide either as a free base or as a pharmaceutically acceptable, non-toxic alkaline salt, such as the $Li^{+}$, $Na^{+}$, $K^{+}$, $Mg^{2+}$, $Ca^{2+}$ or $NH_4^{+}$-salts in combination with a pharmaceutically acceptable carrier. The carrier may be in the form of a solid, semisolid or liquid diluent, or a capsule. The technique to control the release in the abovementioned way has been previously described in EP-A-64 485 as well as in Bogentoft, C. et al., Acta Pharm. Suec., 20, 311-314 (1983): "Delivery of drugs to the colon by means of a new microencapsulated oral dosage form" and Dew, M.J. et al., Br. J. Clin. Pharmac., 14, 405-408 (1982): "An oral preparation to Release Drugs in the Human Colon".

The compounds can also be administered rectally in the form of a pharmaceutical preparation formulated to transport the sulphoxide to the ascending colon, such as a suitably formulated suppository, rectal foam or enema. Usually the amount of active compound is between 2 and 50% by weight of the preparations.

In the preparation of pharmaceutical preparations containing a compound of the present invention in the form of dosage units for oral administration the compound selected may be mixed with a solid, pulverulent carrier, such as lactose, saccharose, sorbitol, mannitol, starch, amylopectin, cellulose derivatives or gelatin, as well as with an anti-friction agent such as magnesium stearate, calcium stearate, and polyethyleneglycol waxes. The mixture is then processed into pellets or tablets. The above prepared cores are coated with a specially formulated coating, which renders the formulation the release characteristics described above. To this coating various dyes may be added in order to distinguish among formulations with different active compounds or with different amount of the active compound present.

Soft gelatine capsules may be prepared, which capsules contain a mixture of the active sulphoxide compound or compounds of the invention and vegetable oil. Hard gelatine capsules may contain granules of the active

sulphoxide compound in combination with a solid, pulverulent carrier as lactose, saccharose, sorbitol, mannitol, potato starch, corn starch, amylopectin, cellulose derivatives or gelatine. The capsules are coated as previously described.

The typical daily dose of the active substance varies within a wide range and will depend on various factors such as for example the individual requirement of each patient, the route of administration and the disease. In general, oral and rectal dosages will be in the range of 1 to 500 mg per day, or more specifically 5-100 mg per day of active substance.

Claims

1. A compound of the formula I

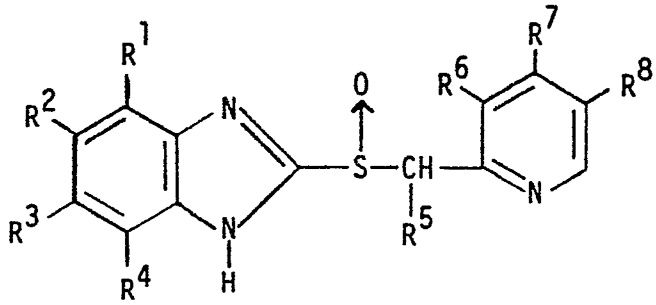

I

or a pharmaceutically acceptable salt thereof for the manufacture of a medicament for treatment of intestinal inflammatory diseases, in which formula I $R^1$, $R^2$, $R^3$ and $R^4$ are the same or different and are

hydrogen
alkyl containing 1-6 carbon atoms
alkoxy containing 1-6 carbon atoms
halogen, e.g. F, Cl, Br, I
$-CF_3$
$-NO_2$
$-COR^9$ ($R^9$ is alkyl or cycloalkyl having 1-6 carbon atoms, aryl containing up to 10 carbon atoms optionally substituted by halogen, methyl or methoxy, or alkoxy containing 1-2 carbon atoms)

or wherein adjacent groups $R^1$, $R^2$, $R^3$ and $R^4$ together with the adjacent carbon atoms in the benzimidazole ring form a 5- or 6-membered ring containing 0-3 hetero atoms selected from N and O and which rings may be either saturated including one or two oxo groups

(-C(=O)-) directly attached to the benzimidazole ring or unsaturated

$R^5$ is hydrogen

$R^6$ and $R^8$, which are the same or different are

hydrogen
methyl or
ethyl

$R^7$ is hydrogen
methyl
methoxy
ethoxy or
aryloxy, wherein the aryl contains up to 10 carbon atoms optionally substituted by alkyl containing 1-6 carbon atoms

provided that

a) when more than one of $R^6$, $R^7$ and $R^8$ are hydrogen and $R^7$ is other than aryloxy, then at least two of $R^1$, $R^2$, $R^3$ and $R^4$ are hydrogen;

b) when $R^7$ is methoxy or ethoxy and $R^6$ and $R^8$ are hydrogen then $R^1$, $R^2$, $R^3$ and $R^4$ are other than CN, $NO_2$ or a 5- or 6-membered ring;

c) when $R^7$ is aryloxy then $R^1$, $R^2$, $R^3$ and $R^4$ cannot be -CN, $-NO_2$, -CO-aryl, $-COOCH_3$, $-COOC_2H_5$ or a 5- or 6-membered ring;

d) when more than one of $R^6$, $R^7$ and $R^8$ are hydrogen and one of $R^1$, $R^2$, $R^3$ and $R^4$ is $CF_3$, then $R^7$ is other than ethoxy;

e) when $R^7$ is methoxy, $R^6$ and $R^8$ are hydrogen and one of $R^1$, $R^2$, $R^3$ and $R^4$ is $CF_3$, then the others of $R^1$, $R^2$, $R^3$ and $R^4$ must be hydrogen.

2. A compound according to claim 1, wherein the compound of the general formula I is

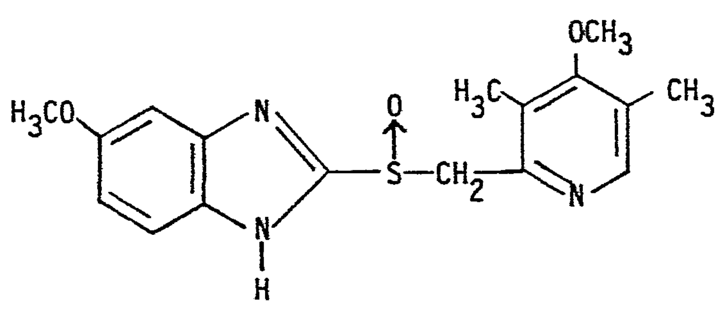

or a pharmaceutically acceptable salt thereof.

3. A compound according to claim 1, for the manufacture of a medicament for treatment of diarrhoea.

0197013

European Patent Office

# EUROPEAN SEARCH REPORT

Application number

EP 86 85 0050

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 045 200 (UPJOHN) * Page 1, lines 4,5 and line 14 - page 2, line 7; page 4, line 36 - page 5, line 25; page 5, line 32 - page 10, line 7; page 8, lines 23-24 * --- | 1,2 | C 07 D 401/12<br>A 61 K 31/415<br>A 61 K 31/44 |
| D,X | DE-A-3 415 971 (BYK GULDEN) * Pages 7-9; page 29, lines 10-17 * --- | 1 | |
| A | DE-A-1 804 450 (EGYESÜLT) --- | | |
| D,E | DE-A- 173 664 (HÄSSLE) * Pages 2-3; page 26, lines 20-21,25 * ----- | 1 | TECHNICAL FIELDS SEARCHED (Int. Cl.4)<br>C 07 D 401/00<br>A 61 K 31/00 |

The present search report has been drawn up for all claims

| Place of search | Date of completion of the search | Examiner |
|---|---|---|
| THE HAGUE | 09-06-1986 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document